# EUROPEAN PATENT APPLICATION

(11) **EP 2 756 806 A1**
(43) Date of publication of application: **23.07.2014**
(21) Application number: 13165061.6
(22) Date of filing: 24.04.2013
(51) Int. Cl.: A61B 8/08, A61B 8/00

(54) **Ultrasound apparatus and method of providing information of the same**

(30) Priority: 16.01.2013 KR 20130005119
(71) Applicant: Samsung Medison Co., Ltd., Gangwon-do 250-870 (KR)
(72) Inventor: Yoo, Jun-sang, Gangwon-do (KR); Choi, Jin-young, Gangwon-do (KR)
(74) Representative: Lorenz, Markus

(57) **Abstract**

Provided are a method of providing information, an ultrasound apparatus, and a recording medium. The method includes obtaining identification information indicating an object, obtaining previously stored guide data that corresponds to the object determined based on the identification information, and providing information about a scan operation to a user by using the obtained guide data.

## Description

### CROSS-REFERENCE TO RELATED PATENT APPLICATION

This application claims the benefit of Korean Patent Application No. 10-2013-0005119, filed on January 16, 2013, in the Korean Intellectual Property Office, the disclosure of which is incorporated herein in its entirety by reference.

### BACKGROUND OF THE INVENTION

### 1. Field of the Invention

The present invention relates to an ultrasound apparatus for providing information about a scan method to a user and a method of providing information of the ultrasound apparatus to a user.

### 2. Description of the Related Art

Ultrasound apparatuses transmit an ultrasound signal (generally equal to or greater than 20 kHz) to a predetermined portion in the interior of an object's body by using a probe and obtain an image of the predetermined portion in the interior of the object's body by using information of an echo signal reflected from the predetermined portion. In particular, ultrasound apparatuses are used for diagnostic and therapeutic purposes, for example, to detect foreign materials in an object or measure or examine an injury. Since such ultrasound apparatuses have advantages of high stability, real-time display, and no risk of X-ray radiation, ultrasound apparatuses are widely used along with other diagnostic imaging apparatuses such as X-ray diagnostic apparatuses, computerized tomography (CT) scanners, magnetic resonance imaging (MRI) apparatuses, and nuclear medicine diagnostic apparatuses.

When an object is diagnosed by using a probe, a user needs to remember a scan position and a scan direction according to a type of the probe and the object. In particular, a resolution of an ultrasound image may vary and the ultrasound image of the object may be shaded, according to a direction of an ultrasound signal emitted to the object from the probe. Accordingly, the user has to remember or know in advance a scan method of effectively scanning the object.

### SUMMARY OF THE INVENTION

According to an aspect of the present invention, there is provided a method of providing information of an ultrasound apparatus, the method including: obtaining identification information indicating an object which a probe is to scan; obtaining guide data that comprises information about a scan operation of an object , wherein the object is determined based on the identification information, and has previously been stored to correspond to the object; and providing the information about the scan operation to a user by using the guide data.

The obtaining of the identification information may include receiving a user input that selects the object and obtaining the identification information based on the user input.

The obtaining of the identification information may include obtaining the identification information based on at least one of ultrasound data of the object which the probe receives and spatial position data of the probe.

The guide data may include information about at least one of a scan position, a scan angle, a scan direction, and a scan axis of the determined object.

The providing may include displaying at least one of a sample image, a sample video, and a notification message for the scan operation.

The providing may include outputting at least one of voice data, a warning sound, and a notification message for the scan operation.

The providing may include scillating the probe for a predetermined period of time.

The providing may include providing along with the information about the scan operation information about an agency or a journal related to the information about the scan operation.

The method may further include displaying along with the information about the scan operation an ultrasound image based on ultrasound data of the object received through the scanned probe.

The method may further include: outputting information about the object, wherein the outputting of information is determined by using the identification information; and receiving a user input that confirms the determined object.

According to another aspect of the present invention, there is provided an ultrasound apparatus including: a probe that scans an object; an object determining unit that obtains identification information indicating the scanned object and determines an object based on the identification information; a storage unit that previously stores, to correspond to the determined object, guide data that includes information about a scan operation of the determined object; and a control unit that obtains the guide data from the storage unit and provides the information about the scan operation to a user by using the guide data.

According to another aspect of the present invention, there is provided a computer-readable recording medium having embodied thereon a program for executing the method.

### BRIEF DESCRIPTION OF THE DRAWINGS

The above and other features and advantages of the present invention will become more apparent by describing in detail exemplary embodiments thereof with reference to the attached drawings in which reference numerals denote structural elements and:
FIG. 1 is a block diagram illustrating an ultrasound apparatus according to an embodiment of the present invention;
FIG. 2 is a flowchart illustrating a method of providing information, according to an embodiment of the present invention;
FIG. 3 is a perspective view for explaining a scan direction of a probe;
FIG. 4 is a view illustrating an example where a scan operation is provided for a tibia and a fibula;
FIG. 5 is a view illustrating an example where a scan operation is provided for a nuchal translucency (NT) and a heart of a fetus;
FIG. 6 is a view illustrating an example where a scan operation is provided for a brain of a newborn baby;
FIG. 7 is a view illustrating an example where a visual notification message or an auditory notification message is output according to a movement of a probe; and
FIG. 8 is a view illustrating an example where an ultrasound image of an object is displayed along with information about a scan operation.

### DETAILED DESCRIPTION OF THE INVENTION

As used herein, the term "and/or" includes any and all combinations of one or more of the associated listed items. Expressions such as "at least one of," when preceding a list of elements, modify the entire list of elements and do not modify the individual elements of the list.

Most of the terms used herein are general terms that have been widely used in the technical art to which the present invention pertains. However, some of the terms used herein may have been created reflecting intentions of technicians in this art, precedents, or new technologies. Also, some of the terms used herein may have been arbitrarily chosen by the present applicant. In this case, these terms are defined in detail below. Accordingly, the specific terms used herein should be understood based on the unique meanings thereof and the whole context of the present invention.

In the present specification, it should be understood that the terms, such as 'including' or 'having', are intended to indicate the existence of the features, numbers, steps, actions, components, parts, or combinations thereof disclosed in the specification, and are not intended to preclude the possibility that one or more other features, numbers, steps, actions, components, parts, or combinations thereof may exist or may be added. Also, the terms, such as 'unit' or 'module', should be understood as a unit that processes at least one function or operation and that may be embodied in a hardware manner, a software manner, or a combination of the hardware manner and the software manner.

The expression "ultrasound image" used herein refers to an image of an object, obtained by using an ultrasound signal. The term 'object' may refer to a part of a body. For example, the object may be an organ such as a liver, heart, nuchal translucency (NT), brain, breast, or belly, or a fetus. Also, the object is not limited thereto, and may be any object that an ultrasound signal obtains ultrasound data of by using a probe.

The ultrasound image may be realized in various ways. For example, the ultrasound image may be at least one of an amplitude (A) mode image, a brightness (B) mode image, a color (C) mode image, and a Doppler (D) mode image. Also, the ultrasound image may be a two-dimensional (2D) image or a three-dimensional (3D) image.

The term "user' used herein may refer to a medical professional such as a doctor, a nurse, a medical laboratory technologist, or a sonographer, but is not limited thereto.

The present invention will now be described more fully with reference to the accompanying drawings for one of ordinary skill in the art to be able to perform the present invention without any difficulty. The invention may, however, be embodied in many different forms and should not be construed as being limited to the embodiments set forth herein; rather, these embodiments are provided so that this disclosure will be thorough and complete, and will fully convey the concept of the invention to those of ordinary skill in the art. Also, parts in the drawings unrelated to the detailed description are omitted to ensure clarity of the present invention. Like reference numerals in the drawings denote like elements.

The present invention will now be described more fully with reference to the accompanying drawings, in which exemplary embodiments of the invention are shown.

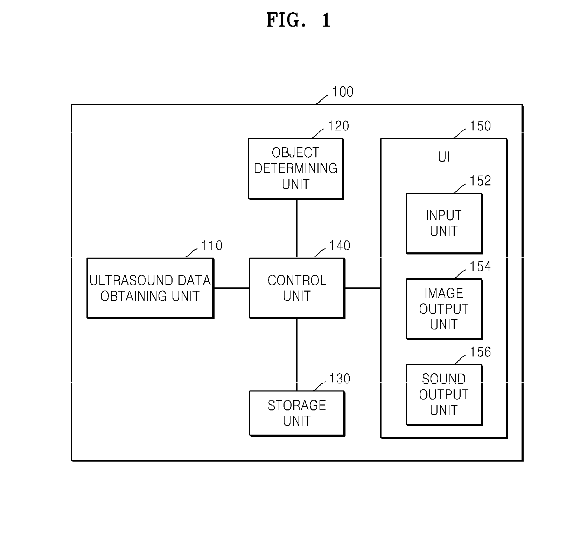

FIG. 1 is a block diagram illustrating an ultrasound apparatus 100 according to an embodiment of the present invention. The ultrasound apparatus 100 may include an ultrasound data obtaining unit 110, an object determining unit 120, a storage unit 130, a control unit 140, and a user interface (Ul) 150. In addition to the elements of FIG. 1, the ultrasound apparatus 100 may further include other general-purpose elements.

The ultrasound apparatus 100 refers to an apparatus that may obtain ultrasound data by scanning an object by using ultrasound, and the ultrasound apparatus 100 may provide information about a scan operation of the object to a user.

The ultrasound apparatus 100 may be embodied in various forms. For example, the ultrasound apparatus 100 may be embodied as a fixed terminal or a mobile terminal. Examples of the mobile terminal may include a picture archiving and communication system (PACS) viewer, a smart phone, a laptop computer, a personal digital assistant (PDA), and a tablet personal computer (PC).

The elements included in the ultrasound apparatus 100 will be successively explained below.

The ultrasound data obtaining unit 110 obtains ultrasound data about the object. The ultrasound data in FIG. 1 may refer to 2D ultrasound data or 3D ultrasound data. Also, the ultrasound data may include Doppler data, which is data indicating a movement of the object.

The ultrasound data obtaining unit 110 of FIG. 1 may include a probe (not shown) for transmitting/receiving an ultrasound signal and a beam former (not shown) for focusing the transmitted/received ultrasound signal. The probe of FIG. 1 may include at least one of a one-dimensional (1D) probe, a 1.5-dimensional (1.5D) probe, and a 2D matrix probe. The ultrasound data obtaining unit 110 may include at least one probe according to an embodiment.

The ultrasound data obtaining unit 110 may directly obtain ultrasound data by scanning the object by transmitting/receiving an ultrasound signal as described above, or may obtain ultrasound data that is previously obtained from another device or an external server.

That is, the ultrasound data obtaining unit 110 may receive ultrasound data in a wired or wireless manner by using at least one element that enables the ultrasound apparatus 100 and an external device to communicate with each other. For example, the ultrasound data obtaining unit 110 may obtain ultrasound data by using a near field communication module, a mobile communication module, a wireless Internet module, or a wired Internet module.

The ultrasound data obtaining unit 110 may obtain ultrasound data in a wired or wireless manner from a hospital server or a cloud server through a medical image information system (e.g., a PACS) as well as the external device.

The object determining unit 120 determines an object that the probe is to scan. That is, the object determining unit 120 may determine an object to which the probe will transmit an ultrasound signal in order to receive an echo signal. For example, the object determining unit 120 may determine any one of various objects that are previously stored, such as a heart, an NT, and a brain.

The object determining unit 120 may obtain identification information, and it may determine an object based on the identification information. The identification information may refer to information indicating an object that the probe is to scan. The identification information may be included in at least one of a user input, ultrasound data, and position data of the probe. That is, the object determining unit 120 may receive not only identification information input by the user, but also identification information from the ultrasound data obtaining unit 110.

Identification information input by the user will be first explained. The object determining unit 120 may obtain identification information based a user input received through an input unit 152 of the UI 150, which will be explained below. That is, the object determining unit 120 may obtain identification information about an object that is being diagnosed, and the obtained information may be received through the input unit 152 from the user who diagnoses the object by using the probe. For example, when the user selects any object from among items of an object list through the input unit 152, the object determining unit 120 may extract identification information about the object from a user input received through the input unit 152.

Identification information received from the ultrasound data obtaining unit 110 will be explained. The object determining unit 120 may obtain identification information based on at least one of ultrasound data received from an object and spatial position data of the probe.

For example, as the probe scans an object, the ultrasound data obtaining unit 110 obtains ultrasound data in real time, and the object determining unit 120 may obtain identification information by analyzing the obtained ultrasound data. That is, when the ultrasound data includes information about the object, the object determining unit 120 may extract identification information from the ultrasound data.

Also, when the ultrasound apparatus 100 includes a plurality of position sensors, the plurality of position sensors may detect a spatial position of the probe. Accordingly, the object determining unit 120 may obtain, from position data indicating a position of the probe grasped by the sensors, identification information indicating an object that is being currently scanned.

Furthermore, the object determining unit 120 may determine an object by using both a user input and ultrasound data. That is, the object determining unit 120 may determine an object based on identification information obtained from ultrasound data and/or position data, and may output information about the determined object through an image output unit 154 and a sound output unit 156. Next, when the input unit 152 receives a user input that confirms or changes the object, the object determining unit 120 may determine an object by further considering the user input.

The storage unit 130 previously stores guide data to correspond to an object. That is, the storage unit 130 may previously store , according to objects, guide data including information about a scan operation regarding each of the objects. The guide data is data that includes not only a scan operation for an object but also general information about a scan method of scanning the object. The storage unit 130 may store the guide data to correspond to each object.

For example, the storage unit 130 may match, to an NT, guide data including information about a scan method of scanning the NT and may store a result of the matching. Likewise, the storage unit 130 may match, to a heart, guide data including information about a method and an operation of scanning the heart and may store a result of the matching.

The guide data may include general information related to scanning of an object as described above. For example, the guide data may include information about at least one of an angle, a direction, and an axis at, in, and along which the probe scans an object. Also, the guide data is not limited thereto, and may include various information related to a scan operation of the object.

The guide data may include information about the scan operation in various forms. For example, the guide data may include visual data such as image data, video data, or popup message data, and auditory data such as voice data, warning sound data, or sound message data. Also, the guide data may include, along with the information about the scan operation, information about an agency or a journal, which is a source of the information about the scan operation.

Furthermore, the guide data may include program information such as a command, a command syntax, or software. For example, the guide data may include program information that controls various elements included in the ultrasound apparatus 100b, such as a command that oscillates the probe for a predetermined period of time or a command that turns on and off a light source provided in the probe.

The storage unit 130 may include at least one storage medium of a flash memory, a hard disk type memory, a multimedia card micro type memory, a card type memory (e.g., secure digital (SD) or extreme digital (XD) memory), a random-access memory (RAM), a static random-access memory (SRAM), a read-only memory (ROM), an electrically erasable programmable read-only memory (EEPROM), a programmable read-only memory (PROM) magnetic memory, a magnetic disk type memory, and an optical disk type memory. Also, the ultrasound apparatus 100 may operate a web storage server or a cloud server that performs a storage function of the storage unit 130 over the Internet.

The control unit 140 provides the information about the scan operation to the user by using the guide data. That is, the control unit 140 may obtain, from the storage unit 130, the guide data about the object as determined by the object determining unit 120 and may provide various information to the user by using the guide data. That is, the control unit 140 may provide various information about the scan operation of the object through the image output unit 154 and the sound output unit 156 included in the UI 150, which will be explained below.

For example, by using the visual guide data, the control unit 140 may display the information about the scan operation and the ultrasound image to the user via the image output unit 154. Also, by using auditory guide data, the control unit 140 may output the information about the scan operation to the user via the sound output unit 156. Also, the control unit 140 may oscillate the probe included in the ultrasound data obtaining unit 110 by using guide data that includes a command for oscillating the probe.

The UI 150 may include the input unit 152 that receives an external input from the user, the image output unit 154 that displays the information about the scan operation and the ultrasound image to the user, and the sound output unit 156 that outputs the information about the scan operation as a sound.

The input unit 152 refers to a unit through which the user inputs information and data for controlling the ultrasound apparatus 100 to the ultrasound apparatus 100. The input unit 152 may receive various control inputs from the user, for example, a touch input.

Examples of the input unit 152 may include, but are not limited to, a keypad, a trackball, a mouse, a dome switch, a touchpad (e.g., a capacitive overlay touchpad, a resistive overlay touchpad, an infrared beam touchpad, a surface acoustic wave touchpad, an integral strain gauge touchpad, or a piezoelectric effect touchpad), a touch panel, a jog wheel, and a jog switch. In particular, the input unit 152 may include a touch screen in which a touchpad and the image output unit 154 are layered.

The touch screen may be configured to detect not only a real touch but also a proximity touch. The expression 'real touch' used herein refers to a case where a pointer touches a screen, and the expression 'proximity touch' used herein refers to a case where the pointer closely approaches the screen but does not touch the screen. The term 'pointer' used herein refers to a tool for touching or closely approaching a specific portion of a displayed screen. For example, the pointer may be a stylus pen or a finger.

Although not shown in FIG. 1, various sensors may be provided inside or outside a touch screen in order to detect a real touch or a proximity touch of the touch screen. For example, a tactile sensor may be used as a sensor for detecting a real touch of the touch screen. The tactile sensor refers to a sensor that detects a touch of a specific object to an extent or more that a human feels. The tactile sensor may detect various information such as a roughness of a contact surface, a stiffness of a touched object, or a temperature of a contact point.

Also, a proximity sensor may be used as a sensor for detecting a proximity touch of the touch screen. The proximity sensor refers to a sensor that uses an electromagnetic force or infrared rays to detect the presence of an object that is nearby to or approaching a predetermined detection surface without any mechanical contact. Examples of the proximity sensor may include a transmissive photoelectric sensor, a direct-reflective photoelectric sensor, a mirror-reflective photoelectric sensor, a high frequency oscillation proximity sensor, a capacitive proximity sensor, a magnetic proximity sensor, and an infrared proximity sensor.

The input unit 152 may receive various touch inputs from the user. Examples of a user input detected by the input unit 152 may include, according to a touch type, a tap, touch and hold, double tap, drag, drag and drop, and swipe.

The image output unit 154 may display and output information processed by the ultrasound apparatus 100. For example, the image output unit 154 may display the ultrasound image of the object on a screen, or display a UI or a graphic UI (GUI) related to function settings.

The image output unit 154 may include at least one of a liquid crystal display, a thin film transistor-liquid crystal display, an organic light-emitting diode, a flexible display, a 3D display, and an electrophoretic display. The ultrasound apparatus 100 may include two or more image output units 154 according to an embodiment of the invention.

The layering of the image output unit 154 and the input unit 154 constitutes the touch screen, and the image output unit 154 may be used as an input device as well as an output device.

The sound output unit 156 outputs information processed by the ultrasound apparatus 100 as a sound. The sound output unit 156 may include a speaker, a buzzer, or the like, and may output various audio data such as voice data or a warning sound.

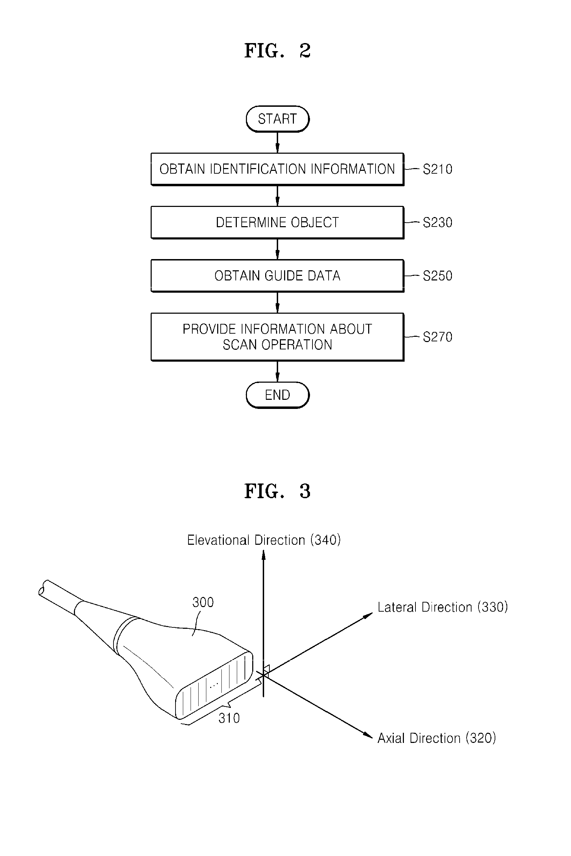

A method of providing information of the ultrasound apparatus 100 by using elements included in the ultrasound apparatus 100 will be explained with reference to FIG. 2. FIG. 2 is a flowchart illustrating a method of providing information, according to an embodiment of the present invention. The method includes operations sequentially performed by the ultrasound data obtaining unit 110, the object determining unit 120, the storage unit 130, the control unit 140, and the UI 150 of the ultrasound apparatus 100 of FIG. 1. Accordingly, although not described, it will be understood that the descriptions previously provided for the elements of FIG. 1 may apply to the method of FIG. 2.

In operation S21 0, the ultrasound apparatus 100 obtains identification information. That is, the ultrasound apparatus 100 may obtain identification information input by a user or may obtain identification information from at least one of ultrasound data and position data of a probe.

The ultrasound apparatus 100 may obtain identification information from a user input that selects any one from among a plurality of items of an object list. That is, the ultrasound apparatus 100 may obtain identification information indicating an object selected by the user. Alternatively, the ultrasound apparatus 100 may obtain identification information indicating an object to be scanned based on at least one of ultrasound data obtained by scanning the object and position data of the probe detected by using a plurality of position sensors.

In operation S230, the ultrasound apparatus 100 determines an object. That is, the ultrasound apparatus 100 may determine an object that the probe scans based on the identification information obtained in operation S210.

In operation S250, the ultrasound apparatus 100 obtains guide data. That is, the ultrasound apparatus 100 may obtain previously stored guide data that corresponds to the object determined in operation S230. As described above, the guide data may include information about a scan operation for the object, for example, information about at least one of a scan position, a scan angle, a scan direction, and a scan axis.

In operation S270, the ultrasound apparatus 100 provides the user with the information about the scan operation. That is, the ultrasound apparatus 100 may display the information about the scan operation included in the guide data or may output the information about the scan operation included in the guided data as a sound, by using the guide data obtained in operation S250. Alternatively, the ultrasound apparatus 100 may guide the scan operation of the user by using various methods, for example, by oscillating the probe or turning on and off a light source based on a command or a program included in the guide data.

Accordingly, since the ultrasound apparatus 100 detects an object and provides information about a scan operation, the user of the ultrasound apparatus 100 does not need to remember a scan operation of each object. That is, when the user performs scanning as guided by the ultrasound apparatus 100, the user of the ultrasound apparatus 100 may efficiently diagnose the object by using an accurate ultrasound image generated by the ultrasound apparatus 100.

FIG. 3 is a perspective view for explaining a scan direction of a probe 300. The probe 300 of FIG. 3 includes a plurality of transducers 310, and the probe 300 transmits an ultrasound signal by controlling the plurality of transducers 310. A user of the ultrasound apparatus 100 scans an object by moving the probe 300. A scan direction in which the probe 300 is moved will be explained in detail.

A direction in which an ultrasound signal is transmitted from the probe 300 is referred to as an axial direction 320. A direction in which the plurality of transducers 310 of the probe 300 are arranged is referred to as a lateral direction 330. Although the probe 300 has a linear array of transducers for convenience of explanation, the probe 300 may have a phased array or convex array of transducers. A direction in which the probe 300 is lifted up is referred to as an elevational direction 340.

Information about such a scan direction may be included in guide data described with reference to FIGS. 1 and 2. That is, the ultrasound apparatus 100 may previously store information about a scan direction, which is information about a direction in which a probe is moved to diagnose an object, in guide data.

The guide data may include information about at least one of a scan position, a scan direction, a scan angle, and a scan axis as described with reference to FIGS. 1 and 2.

The expression 'scan position' refers to a position at which a probe is closely attached to a human body in order to diagnose a desired object. That is, the scan position may refer to a proximity position of a probe for efficiently obtaining ultrasound data of an object.

The expression 'scan angle' refers to an angle between a probe and an object. That is, when the scan angle is 90 degrees, it means that a probe is located adjacent to an object such that a direction of an ultrasound signal emitted from the probe is perpendicular to the object.

The expression 'scan axis' refers to a direction in which transducers are arranged. That is, in FIG. 3, the plurality of transducers 310 are arranged in a horizontal direction. When an object is diagnosed, the user may approach the probe 300 to the object by rotating the probe 300 by 90 degrees such that the transducers 310 are arranged in a vertical direction. That is, information about a scan axis may refer to information about a direction, from among a horizontal direction and a vertical direction, in which a user approaches a probe to an object to diagnose the object.

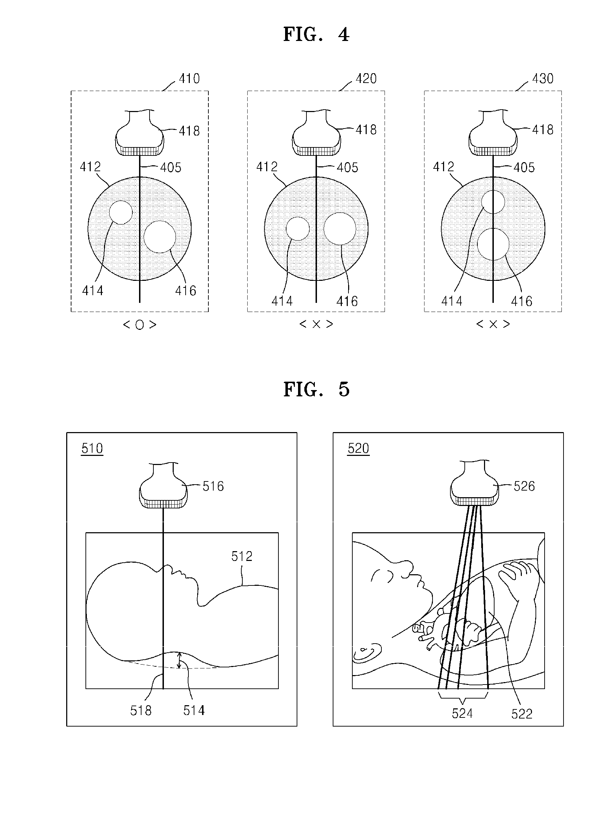

FIG. 4 is a view illustrating an example where a scan operation is provided for a tibia and a fibula. In FIG. 4, a dark circle 412 indicates a calf. From among two circles included in the circle 412, a smaller circle 414 indicates a fibula and a larger circle 416 indicates a tibia. A user of the ultrasound apparatus 100 may diagnose the fibula 414 and the tibia 416 by approaching the probe 418 to the calf 412 and scanning the calf 412.

Before explaining an operation of the ultrasound apparatus 100 of FIG. 4, a clinical explanation will be first made. When a tibia and a fibula are diagnosed by using ultrasound, an accuracy of an ultrasound image varies according to a scan operation such as a scan position or a scan axis. For example, when an axial direction 405 of the probe 418 is perpendicular to a line connecting the tibia 416 and the fibula 414 as shown in a sample image 420, an ultrasound image may be generated in which two bones, that is, the tibia 416 and the fibula 414, look like they are joined together. When the axial direction 405 of the probe 418 passes through the tibia 416 and the fibula 414 as shown in a sample image 430, a deeper bone may be shaded, and thus an accurate measurement result may not be obtained.

Accordingly, as shown in a sample image 410, the user diagnoses the object by approaching the probe 418 to the calf 412 such that the axial direction 405 is oblique to a line connecting the tibia 416 and the fibula 414.

When the tibia 416 and the fibula 414 are determined as an object, the ultrasound apparatus 100 may obtain identification information based on at least one of a user input, ultrasound data, and position data of the probe 418, and may determine that an object is the tibia 416 and the fibula 414 according to the identification information.

Next, the ultrasound apparatus 100 obtains guide data that is previously stored for the tibia 416 and the fibula 414. The guide data may include the sample images 410, 420, and 430 of FIG. 4, and may include various data such as video data, audio data, and a program described with reference to FIGS. 1 and 2.

The ultrasound apparatus 100 provides the information about the scan operation to the user by using the guide data. In FIG. 4, the ultrasound apparatus 100 may provide the information about the scan operation to the user by displaying at least one of the sample images 410, 420, and 430. As described above, the sample image 410 is an image indicating a scan position at which the tibia 416 and the fibula 414 may be accurately measured, and the sample image 420 and the sample image 430 are scan positions at which inefficient or inaccurate results are predicted.

For example, the ultrasound apparatus 100 may provide information to guide the user to accurately diagnose an object by displaying only the sample image 410. Alternatively, the ultrasound apparatus 100 may also provide information to the user about a case where inaccurate diagnostic results may be obtained, by displaying the sample image 420, the sample image 430 and the sample image 410.

Although the sample images 410 through 430 are illustrated in FIG. 4, the guide data may use an ultrasound image obtained by based on actual images of the tibia 416 and the fibula 414 as a sample image. Also, the ultrasound apparatus 100 may provide to the user video data showing a process of diagnosing the tibia 416 and the fibula 414 as described above.

FIG. 5 is a view illustrating an example where a scan operation is provided for an NT 514 of a fetus 512 and a heart 522 of the fetus 512.

A sample image 510 is an image indicating a scan operation for diagnosing the NT 514 of the fetus 512. In order to accurately measure a thickness of the NT 514, an axial direction 518 of a probe 516 has to pass through a thickest portion of the NT 514.

A sample image 520 is an image indicating a scan operation for diagnosing the heart 522 of the fetus 512. The heart 522 may be diagnosed based on various protocols 524 such as a 4 chamber view, a 5 chamber view, and a trachea and vessel view according to a scan position. A scan position and a scan angle may vary according to the protocols 524.

In FIG. 5, the ultrasound apparatus 100 considers the NT 514 and the heart as separate objects, and obtains guide data that is stored to correspond to each object. Next, the ultrasound apparatus 100 may output information about a scan operation for the NT/heart, and may guide a user. The information about the scan operation may include visual information, auditory information, or tactile information, such as an oscillation of the probe 516 or 526 as described with reference to FIG. 4.

The ultrasound apparatus 100 may provide, along with the information about the scan operation, information about an agency or a journal related to the scan operation. For example, when information about a heart protocol of the fetus 522 is provided to the user as shown in the sample image 520, the ultrasound apparatus 100 may improve reliability of the information of the scan operation by outputting information about an agency, a journal, an author, or the like, which is a source of the heart protocol, as a text or an image.

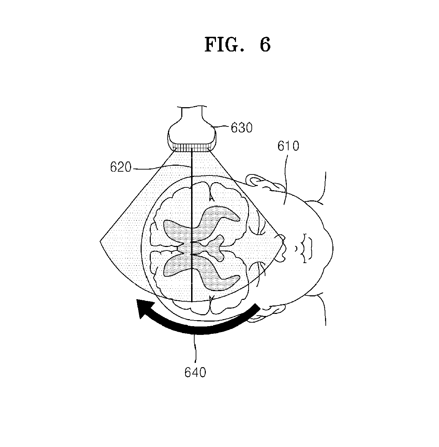

FIG. 6 is a view illustrating an example where a scan operation is provided for a brain of a newborn baby 610. As described with reference to FIGS. 4 and 5, the ultrasound apparatus 100 may previously store a scan operation for accurately and efficiently diagnosing a brain, in which the previously stored scan operation corresponds to the brain, and in which the brain is an object. When the brain is determined as an object, the ultrasound apparatus 100 provides information about the scan operation to a user by using stored guide data.

The scan operation for the brain may be an operation of moving a probe 630 in an axial direction 620 toward a pate (head crown) from the eyes of the newborn baby 610. Accordingly, the ultrasound apparatus 100 may alert the user of a change in a scan direction of the probe 630 by displaying an arrow 640 as shown in FIG. 6. Alternatively, the ultrasound apparatus 100 may display a video showing that the probe 630 is moved in a direction indicated by the arrow 640.

When the ultrasound apparatus 100 includes a plurality of sensors that detect a spatial position of the probe 630, the ultrasound apparatus 100 may detect a change in a scan direction of the probe 630. That is, when the user scans the brain, which is an object, by moving the probe 630, the ultrasound apparatus 100 may detect a movement of the probe 630 by using the position sensors.

For example, when the probe 630 scans the brain by being moved according to a change in a previously stored scan direction, the ultrasound apparatus 100 may display a notification message or output a sound indicating that the scan operation is being performed correctly. By contrast, when the probe 630 scans the brain by being moved in a direction different from a previously stored scan direction, the ultrasound apparatus 100 may provide information to the user indicating that the scan operation is being performed differently from that which has been previously stored.

Thus, when the scan operation is performed differently from that which is previously stored, the ultrasound apparatus 100 may inform the user that the scan operation has to be performed again, by slightly oscillating the probe 630 or by turning on and off at least one light source provided in the probe 630. That is, the ultrasound apparatus 100 may provide tactile information or visual information by controlling the probe 630 by using a program or a command included in the guide data. The ultrasound apparatus 100 may provide to the user various information such as synesthesia information as well as the visual, auditory, and tactile information.

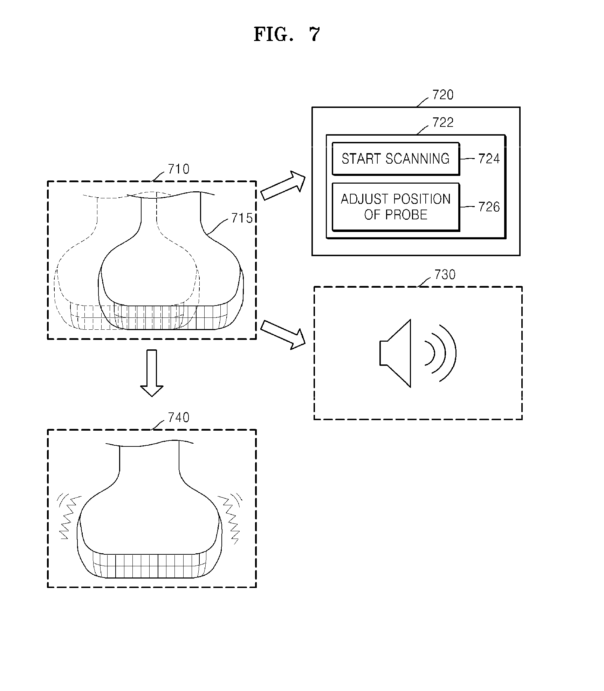

FIG. 7 is a view illustrating an example where a visual notification message, an auditory notification message, or a tactile notification message is output according to a movement of a probe 715.

The ultrasound apparatus 100 may detect a position at which the probe 715 is closely attached to an object in order to diagnose the object. That is, as shown in the left of FIG. 7, the ultrasound apparatus 100 may detect that the probe 715 is moved from a position marked by a dashed line to a position marked by a solid line.

Next, when it is detected that the probe 715 is moved to a scan position included in guide data, the ultrasound apparatus 100 may display a notification message on a screen 720 as shown in the top right of FIG. 7. In FIG. 7, the ultrasound apparatus 100 provides information about a scan operation to a user by displaying a popup window 722.

That is, the ultrasound apparatus 100 may use the popup window 722 to inform the user that the probe 715 has been moved to a position that corresponds to a previously stored scan position, and may display a menu item 726 for adjusting a position of the probe 715 and a menu item 724 for starting a scan. The user may conveniently diagnose the object by selecting the menu item 724 for starting a scan, or the user may readjust a position of the probe 715 by referring to the information about the scan operation that is displayed on the screen 720.

Alternatively, as shown in an image 730 of FIG. 7, the ultrasound apparatus 100 may output a notification message as a sound. That is, the ultrasound apparatus 100 may output a notification message by using audio data that is previously stored, and the output notification message may indicate that a scan position is accurate or that a scan position needs to be adjusted.

Alternatively, as shown in an image 740 of FIG. 7, the ultrasound apparatus 100 may output a tactile notification message that oscillates the probe 715. A length of time that the ultrasound apparatus 100 oscillates the probe 715 and the intensity of the oscillation may be adjusted according to a user input and may be previously stored in the ultrasound apparatus 100.

When it is detected that the probe 715 is out of a position at which the probe 715 may scan the object, the ultrasound apparatus 100 may oscillate the probe 715 to inform the user that a position of the probe 715 needs to be adjusted.

By contrast, assuming that the user moves the probe 715 in order to scan the object, when it is detected that the probe 715 reaches an appropriate position at which the probe 715 may scan the object, the ultrasound apparatus 100 may oscillate the probe 715. Accordingly, the ultrasound apparatus 100 may guide the user in the diagnosis of the object.

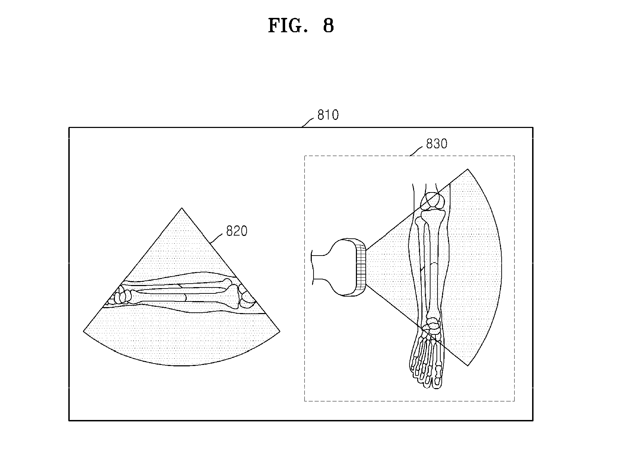

FIG. 8 is a view illustrating an example where an ultrasound image of an object is displayed along with information about a scan operation. In FIG. 8, the example of FIG. 7 using the tibia 416 and the fibula 414 is illustrated.

The ultrasound apparatus 100 may visually provide information 830 about a scan operation for a tibia and a fibula on a screen 810. That is, the ultrasound apparatus 100 may visually output information about at least one of a scan position, a scan direction, a scan angle, and a scan axis of a probe for scanning the tibia and the fibula. As described above, the ultrasound apparatus 100 may output auditory information such as a sound, or tactile information that oscillates the probe.

The ultrasound apparatus 100 may display an ultrasound image 820 generated when the probe scans the object along with the information 830 about the scan operation. Accordingly, the ultrasound apparatus 100 may output a sample image that the user wishes to obtain and an actually measured image, and the user may compare the sample image with the actually measured image to determine whether the object has been accurately measured. That is, the ultrasound apparatus 100 may use the information 830 about the scan operation as a body marker.

The present invention may be embodied as a program executed in a computer, and may be implemented in a general purpose digital computer by using a computer-readable medium. Also, a structure of data used in the method may be recorded by using various units on a computer-readable medium. It should be understood that program storage devices, as may be used to describe storage devices containing executable computer code for operating various methods of the present invention, shall not be construed to cover transitory subject matter such as carrier waves or signals. Examples of the computer readable recording medium include magnetic storage media (e.g., ROM, floppy disks, hard disks, etc.), optical recording media (e.g., CD-ROMs, or DVDs), etc.

According to an ultrasound apparatus, a method of providing information, and the recording medium of the present invention, a user of the ultrasound apparatus does not have to know in advance information about a scan operation of each object. That is, since previously stored information about a scan operation and a scan method is automatically provided, an accurate ultrasound image may be obtained and an object may be efficiently diagnosed.

Also, dependence on the user's skill may be reduced, and the risk of diagnostic error or misdiagnosis may be reduced. Accordingly, a learning curve for ultrasound diagnosis may be reduced, and the user may easily and conveniently use the ultrasound apparatus.

While the present invention has been particularly shown and described with reference to exemplary embodiments thereof by using specific terms, the embodiments and terms have merely been used to explain the present invention and should not be construed as limiting the scope of the present invention as defined by the claims. The exemplary embodiments should be considered in a descriptive sense only and not for purposes of limitation. Therefore, the scope of the invention is defined not by the detailed description of the invention but by the appended claims, and all differences within the scope will be construed as being included in the present invention.

## Claims

1. A method of providing information of an ultrasound apparatus, the method comprising:
obtaining identification information indicating an object that is to be scanned by a probe;
obtaining guide data that comprises information about a scan operation of the object, wherein the object is determined based on the identification information, and the obtained guide data has previously been stored and corresponds to the object; and
providing the information about the scan operation to a user by using the obtained guide data.

2. The method of claim 1, wherein the obtaining of the identification information comprises receiving a user input that selects the object that is to be scanned by the probe, and obtaining the identification information based on the user input.

3. The method of claim 1, wherein the obtaining of the identification information is based on at least one of ultrasound data received by the probe and spatial position data of the probe.

4. The method of claim 1, wherein the obtained guide data comprises information about at least one of a scan position, a scan angle, a scan direction, and a scan axis of the determined object.

5. The method of claim 1, wherein the providing comprises displaying at least one of a sample image, a sample video, and a notification message for the scan operation.

6. The method of claim 1, wherein the providing comprises outputting at least one of voice data, a warning sound, and a notification message for the scan operation.

7. The method of claim 1, wherein the providing comprises oscillating the probe for a predetermined period of time.

8. The method of claim 1, wherein the providing comprises providing along with the information about the scan operation, information about an agency or a journal related to the information about the scan operation.

9. The method of claim 1, further comprising displaying along with the information about the scan operation, an ultrasound image received through the probe based on ultrasound data of the scanned object.

10. The method of claim 1, further comprising:
outputting information about the object, wherein the outputting of information is determined based on the obtained identification information; and
receiving a user input that confirms the determined object.

11. An ultrasound apparatus comprising:
a probe that scans an object;
an object determining unit that obtains identification information indicating the scanned object and determines an object based on the obtained identification information;
a storage unit that previously stores, to correspond to the determined object, guide data that comprises information about a scan operation of the determined object; and
a control unit that obtains the guide data from the storage unit and provides the information about the scan operation to a user by using the obtained guide data.

12. The ultrasound apparatus of claim 11, further comprising an input unit that receives a user input that selects the object that is to be scanned by the probe,
wherein the object determining unit obtains the identification information based on the user input.

13. The ultrasound apparatus of claim 11, further comprising an ultrasound data obtaining unit that receives at least one of ultrasound data of the scanned object and spatial position data of the probe,
wherein the object determining unit obtains the identification information based on at least one of the ultrasound data of the scanned object and the spatial position data.

14. The ultrasound apparatus of claim 11, further comprising:
an image output unit that outputs information about the determined object, wherein the outputted information is determined by using the identification information; and
an input unit that that receives a user input that confirms the determined object.

15. A non-transitory computer-readable recording medium having embodied thereon a program, which, when executed by a computer, performs the method of claim 1.
